# EUROPÄISCHE PATENTSCHRIFT

(11) **EP 0 596 829 B1**
(45) Veröffentlichungstag und Bekanntmachung des Hinweises auf die Patenterteilung: **22.11.2000**
(21) Anmeldenummer: 93810575.6
(22) Anmeldetag: 12.08.1993
(51) Int. Cl.: A61F 2/08

(54) **Verankerung für ein künstliches Band**
Anchor for synthetic ligament
Ancrage pour ligament synthétique

(30) Priorität: 02.11.1992 EP 92810838
(43) Veröffentlichungstag der Anmeldung: 11.05.1994
(73) Patentinhaber: Sulzer Orthopädie AG, 6340 Baar (CH)
(72) Erfinder: Steininger, Roland, Dr., CH-8400 Winterthur (CH); Bivi, Luigi, CH-4051 Basel (CH)
(74) Vertreter: Sulzer Management AG

(56) Entgegenhaltungen:
- EP-A- 0 330 328
- EP-A- 0 465 408
- FR-A- 2 586 927
- FR-A- 2 676 356
- ARTHROSCOPY Bd. 5, Nr. 3 , September 1989 , NEW YORK US Seiten 225 - 226 MATTHEWS ET AL. 'Pitfalls in the use of interference screws for anterior cruciate ligament reconstruction'

## Beschreibung

Die Erfindung handelt von einer Verankerung für ein künstliches Band, welches durch einen Knochen hindurchgeführt ist, wobei die Verankerung aus einer im Knochen abgestützten Hülse und aus einem Klemmelement mit Geschossform besteht, und wobei in einem Schenkel der Hülse ein Band einziehbar ist, während quer dazu das Klemmelement verstellbar ist, um eine Klemmkraft auf das eingezogene Band aufzubringen.

In der EP-A 0 465 408 ist eine einfache Bandverankerung mit einer konischen Klemmhülse gezeigt, welche selbsthemmend in einer konischen Verankerungshülse gelagert ist. Ähnlich zeigen FR-A 2 586 927 und EP-A 0 232 049 konische Klemmelemente, die in einen strumpfartigen Bandfortsatz eingeschoben werden, um gegen eine Verankerungshülse zu klemmen. Diese Verankerungshülse kann ihrerseits nachgespannt werden, was zusätzliche Elemente erfodert, oder es wird beim Operateur einiges Geschick vorausgesetzt, um die gegenläufigen Bewegungen von Bandstreckung und Klemmen so zu koordinieren, dass sich das Band mit der gewollten Vorspannung festsetzt.

Im weiteren zeigt die EP-A 0 330 328 eine Verankerung für ein Kreuzband, bei der durch einen Achsversatz eine Schrägschulter in der Aufnahmebohrung für das Band erzeugt wird und indem mit der verrundeten Spitze einer quer zur Achse angebrachten Madenschraube das Band direkt, oder indirekt über eine deformierbare Zwischenhülse, zwischen Spitze und Schrägschulter verpresst wird. Für eine dauerhafte Fixierung des Bandes muss die Reibung ausreichen, welche durch Pressung zwischen Spitze und Schrägschulter erzeugt wird. Unvorsichtiges Spannen führt zu Druckspitzen, die das Bandmaterial schädigen.

In der FR-A-2 676 356 ist ein Verankerungskörper in Form eines Kegelstumpfes gezeigt. Der Kanal durch den das künstliche Band eingezogen ist besitzt eine Aussparung, um eine zu einer Klemmschraube senkrecht stehende Anpressfläche und durch eine Umlenkung des ganzen Bandes in eine eigentliche Zugrichtung eine Erhöhung der Rückhaltekraft zu erreichen. Die Klemmschraube bohrt sich mit ihrer Spitze in das Band, um es in dem Kanal zu verriegeln. Eine derartige konzentrierte Belastung des Bandes ist nicht besonders schonend.

Aufgabe der Erfindung ist es, mit einfachen Mitteln eine Verankerung des implantierten Bandes bei einer vorgesehenen Vorspannung sicher zu erreichen. Die Lösung dieser Aufgabe wird mit den Merkmalen des unabhängigen Hauptanspruchs 1 erreicht, indem die Hülse gegen aussen als Hosenrohr ausgeführt ist, dessen Schenkel in einem spitzen Winkel zwischen 40° und 20° zueinanderstehen, indem sich die Rohrinnenflächen des ersten Schenkels für das Band und des zweiten Schenkels für das Klemmelement gegenseitig auf der ganzen Länge der Verzweigung durchdringen, und indem sowohl das Klemmelement als auch der erste Schenkel Rillen quer zum Band aufweisen, welche das Band gemeinsam auf einer Länge von mehr als dem Innendurchmesser des ersten Schenkels auf 360° umgeben und verpressen, wobei die Klemmung mit einer Seitenfläche der langgezogenen Geschossform erfolgt.

Die Lösung hat den Vorteil, dass die Klemmkraft unabhängig von der Vorspannung des Bandes von aussen einstellbar ist, wobei wegen der grossen und strukturierten Klemmfläche ein lokales Zerstören durch Druckspitzen verhindert wird. Die Strukturierung der Klemmfläche entlang einer grösseren Länge des Bandes verhindert ein Wegfliessen desselben. Durch Verwendung einer Hülse, die als Hosenrohr ausgeführt ist, ergibt sich eine einfache Vorbearbeitung im Knochen, indem die Durchtrittsöffnung für das Band auf den Durchmesser eines ersten Schenkels erweitert wird und im Winkel des dazu stehenden zweiten Schenkels eine zweite Bohrung angebracht wird. Das Band kann in der im Knochen eingesetzten Hülse durch den ersten Schenkel gezogen werden, und beispielsweise mit einer Federwaage unter Vorspannung gehalten werden, während die Klemmung unabhängig durch ein Klemmelement im zweiten Schenkel erfolgt. Das Band kann von einem Endlosstapel in der passenden Länge eingezogen werden. Nach dem Klemmen und Abschneiden des Bandes schliesst die Verankerung bündig mit der Knochenoberfläche ab.

Für die Verankerung von Kreuzbändern auf der Tibiaseite ist es vorteilhaft, wenn gemessen in der Ebene des Winkels zwischen den Schenkeln die Stirnseite der Hülse einen Neigungswinkel von weniger als 80° zum ersten Schenkel aufweist. Dabei kann zur stabileren Verankerung einer der beiden Schenkel über den Schnittpunkt der beiden Schenkelachsen hinaus in seiner Richtung fortgesetzt sein. Um der Hülse mehr Halt in Zugrichtung zu geben, besitzt mindestens der fortgesetzte Schenkel, über den der Bandzug erfolgt, in der Ebene der Stirnfläche einen vorstehenden Lappen. Die Spitze des Klemmelements ist patronenförmig und gerillt ausgeführt, um im Zusammenwirken mit dem spitzen Winkel zwischen den Schenkeln eine Anpressfläche zu erzeugen, die sich über eine grössere Länge des Bandes erstreckt. Dabei kann das Klemmelement einmal als eine im zweiten Schenkel durch Drehung stufenlos verstellbare Klemmschraube ausgeführt sein oder hinter seiner Spitze als mehrfach längsgeschlitzte Hülse ausgeführt sein, deren Segmente über Sägezahnrillen in einer Gegenrillung des zweiten Schenkels verankerbar sind. Um die Segmente von einer dauernden Biegebelastung zu entlasten, kann nach der Festsetzung des Bandes im Kernbereich der mehrfach längsgeschlitzten Hülse ein Stift eingesetzt werden.

Im folgenden ist die Erfindung anhand von Ausführungsbeispielen beschrieben. Es zeigen:
- Fig. 1: die Ansicht einer Hülse ohne Klemmelement;
- Fig. 2: schematisch den Schnitt durch eine im Knochen eingesetzte Bandverankerung, die als Klemmelement eine Klemmschraube aufweist;
- Fig. 3: schematisch den Schnitt durch eine im Knochen eingesetzte Bandverankerung, die einen Klemmkörper mit einer geschlitzten Hülse und mit gezahnten Biegefedern aufweist; und
- Fig. 4: schematisch den Querschnitt durch eine geschlitzte Hülse mit gezahnten Biegefedern nach Figur 3.

In den Figuren sind Verankerungen für ein künstliches Band wie z.B. ein künstliches Kreuzband gezeigt, welches durch einen Knochen hindurchgeführt ist. Die Verankerung besteht aus einer im Knochen abgestützten Hülse, die gegen aussen als Hosenrohr ausgeführt ist, dessen Schenkel in einem spitzen Winkel zueinanderstehen. In einem ersten Schenkel ist das künstliche Band einziehbar, während im zweiten Schenkel ein Klemmelement in Richtung der Schenkelachse verstellbar ist, um mit seiner gerillten Mantelfläche das Band an der gerillten Innenfläche des ersten Schenkels zu fixieren.

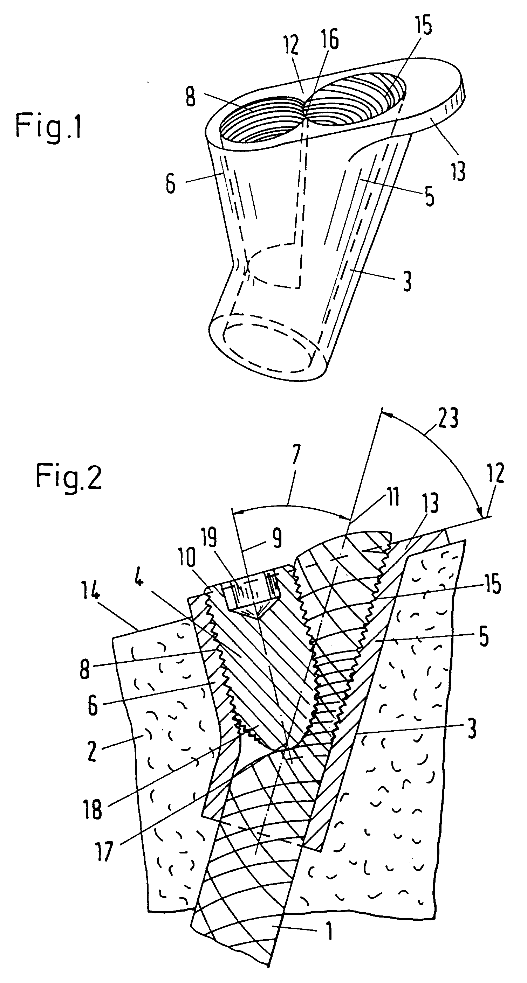

In Figur 1 ist eine Hülse 3 in Form eines Hosenrohrs gezeigt, dessen Schenkel 5, 6 soweit gekürzt sind, dass ein Zwickel wegfällt, und dass die Rohrinnenflächen der Schenkel 5, 6 sich gegenseitig durchdringen resp. in einem Durchbruch ineinander übergehen. Ein erster Schenkel 5 ist in Richtung seiner Schenkelachse weiter fortgesetzt. Seine Innenfläche ist im Bereich gegenüber dem im spitzen Winkel 7 wegstehenden zweiten Schenkel 6 mit Querrillen 15 versehen. An seiner Aussenseite ist ein in der Ebene 12 der Stirnfläche der Hülse 3 vorstehender Lappen 13 angebracht. Der zweite Schenkel hat auf seiner Rohrinnenseite ein Gewinde 8 das als Abstützung für ein Klemmelement 4 in Form einer Klemmschraube 10 vorgesehen ist.

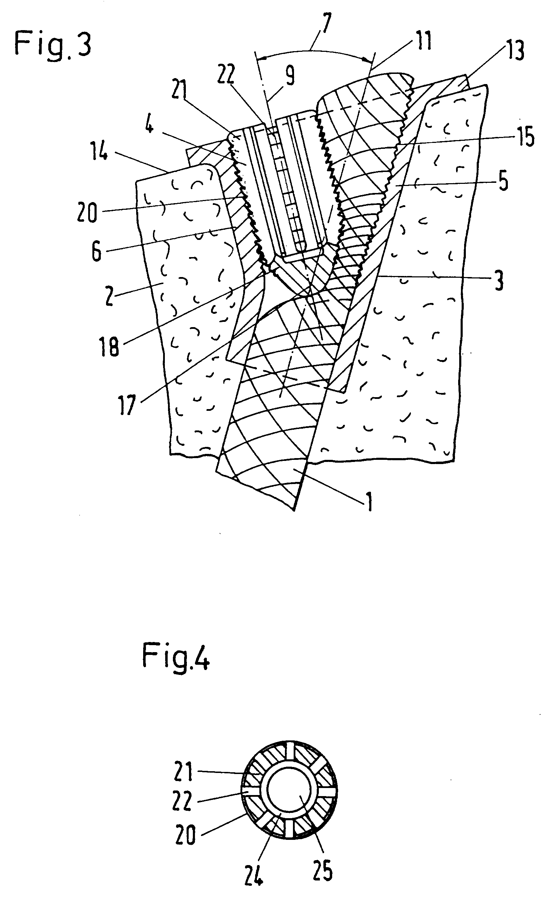

Figur 2 zeigt die gleiche Hülse 3 im Knochen 2 eingepasst mit einem durch den Knochen eingezogenen künstlichen Band 1. Die beiden Schenkel 5, 6 stehen in einem Winkel 7 zwischen 40° und 20° zueinander und haben einen durchgehenden Durchbruch 16 zueinander, indem sie sich gegenseitig durchdringen. Gemessen in der Ebene der beiden Schenkelachsen 11 und 9 steht die Ebene 12 der Stirnfläche der Hülse 3 in einem Neigungswinkel 23 von weniger als 80° zur Schaftachse 11 des ersten Schenkels 5, welcher über den Schnittpunkt 17 der beiden Schenkelachsen 11, 9 hinaus in seiner Richtung fortgesetzt ist. Im zweiten Schenkel 6 ist ein durch den Durchbruch 16 unterbrochenes Gewinde 8 angebracht, auf dem sich ein Klemmelemnt 4 mit Geschossform 18, als Klemmschraube 10 über einen Innensechskant 19 bewegbar, mit einem Aussengewinde abstützt. Die Klemmung des eingezogenen Bandes 1 erfolgt mit einer Seitenfläche der langezogenen Geschossform der Klemmschraube 10, die das Band 1 seitlich gegen die Wand des ersten Schenkels 5 presst und eine langgezogene Druckzone erzeugt. Zur Unterstützung der Haftung des Bandes 1 sind die Querrillen 15 bis in die Druckzone hineingeführt und können scharfe Kanten aufweisen. Die Haftung wird durch den im Durchbruch 16 am Band 1 anliegenden Gewindeteil der Klemmschraube 10 auf 360° erhöht. Die Querrillen 15 können auch eine leichte Neigung aufweisen, wenn sie wie ein Innengewinde mit einem Gewindebohrer erzeugt werden. Mit der Klemmschraube 10 ist auch an einem vorgespannten Band 1 eine stufenlose Einstellung der Klemmkraft möglich. Eine weitere Abstützung des Klemmelementes 4 ist in Figur 4 und 5 gezeigt. Der hintere Teil des geschossförmigen Körpers hat die Form einer mehrfach längsgeschlitzten Hülse mit zylindrischem Hohlraum 24. Längsschlitze 22 unterteilen die Mantelfläche in Biegefedern 21. Diese weisen in Längsrichtung auf ihrer Aussenseite eine Sägezahnung 20 auf, welche unter der Vorspannung der Biegefedern 21 an einer Gegenzahnung der Innenfläche des zweiten Schenkels 6 anliegt. Die Sägezahnung ist so ausgelegt, dass sie selbsthemmend gegen ein Rückwärtsgleiten des Klemmelementes 4 wirkt. Zum Klemmen des Bandes muss der Klemmkörper 4 von Einraststation zu Einraststation vorwärtsgetrieben werden, bis die notwendige Klemmkraft erreicht ist. Dies geschieht beispielsweise mit einem Stössel (hier nicht gezeigt), der am Boden 25 des zylindrischen Hohlraums 24 aufliegt und einen kleineren Durchmesser als der letztere aufweist, damit sich die Biegefedern 21 beim Einfahren des Klemmelementes 4 zur Längsachse hin deformieren können. Wenn die Biegefedern 21 relativ schwach ausgelegt werden, kann nach dem Erreichen der Klemmstellung ein Sicherungsstift oder eine Sicherungsschraube im zylindrischen Hohlraum 24 befestigt werden, um ein Rückfedern oder Ermüden der Biegefedern 21 zu verhindern.

Bei eingesetzter Verankerung wirkt die Zugkraft des künstlichen Bandes 1 in der Richtung der Schenkelachse 11 des ersten Schenkels 5. Als wirksame Flächen für eine Kraftübertragung auf den Knochen 2 sind die über den Innendurchmesser des ersten Schenkels 5 vorstehende und auf einer zur Schenkelachse 11 senkrecht stehende Ebene projezierte Flächen anzusehen. Es sind dies der Lappen 13 und die projezierte Fläche vom zweiten Schenkel 6. Falls diese Fläche als zu gering betrachtet wird, kann der Lappen 13 wie in Figur 3 ausgedeutet bis auf die Gegenseite in der Ebene 12 herumgeführt werden.

Für die Verankerung stehen dem Operateur mehrere Hülsen 3 zur Verfügung, die sich durch unterschiedliche Neigungswinkel 23 der Ebene 12 ihrer Stirnfläche zur Schaftachse 11 unterscheiden. Er kann somit die Verankerung an die Zugrichtung des künstlichen Bandes 1 anpassen.

## Patentansprüche

1. Verankerung für ein künstliches Band (1), welches durch einen Knochen (2) hindurchgeführt ist, wobei die Verankerung aus einer im Knochen (2) abgestützten Hülse (3) und aus einem Klemmelement (4) mit Geschossform (18) besteht, und wobei in einem Schenkel (5) der Hülse (3) ein Band (1) einziehbar ist, während quer dazu das Klemmelement (4) verstellbar ist, um eine Klemmkraft auf das eingezogene Band (1) aufzubringen, wobei die Hülse (3) gegen aussen als Hosenrohr ausgeführt ist, dessen Schenkel (5, 6) in einem spitzen Winkel (7) zwischen 40° und 20° zueinanderstehen, sich die Rohrinnenflächen des ersten Schenkels (5) für das Band und des zweiten Schenkels (6) für das Klemmelement (4) gegenseitig auf der ganzen Länge der Verzweigung durchdringen und sowohl das Klemmelement (4) als auch der erste Schenkel (5) Rillen (8, 15, 20) quer zum Band (1) aufweisen, welche das Band (4) gemeinsam auf einer Länge von mehr als dem Innendurchmesser des ersten Schenkels (5) auf 360° umgeben und verpressen, wobei die Klemmung mit einer Seitenfläche der langgezogenen Geschossform (18) erfolgt.

2. Verankerung nach Anspruch 1, dadurch gekennzeichnet, dass gemessen in der Ebene des Winkels (7) die Stirnseite der Hülse (3) einen Neigungswinkel (23) von weniger als 80° zum ersten Schenkel (5) aufweist.

3. Verankerung nach Anspruch 1 oder 2, dadurch gekennzeichnet, dass einer der beiden Schenkel (5, 6) über den Schnittpunkt der beiden Schenkelachsen (9, 11) in seiner Richtung fortgesetzt ist.

4. Verankerung nach Anspruch 3, dadurch gekennzeichnet, dass mindestens der fortgesetzte Schenkel an seiner Aussenseite in einer Ebene (12) der Stirnfläche der Hülse (3) einen vorstehenden Lappen (13) zur Abstützung an der Knochenaussenseite (14) aufweist.

5. Verankerung nach einem der Ansprüche 1 bis 4, dadurch gekennzeichnet, dass das Klemmelement (4) eine im zweiten Schenkel (6) durch Drehung stufenlos verstellbare Klemmschraube (10) ist mit einer patronenförmigen gerillten Spitze.

6. Verankerung nach einem der Ansprüche 1 bis 4, dadurch gekennzeichnet, dass das Klemmelement (4) hinter einer patronenförmigen Spitze als geschlitzte Hülse ausgeführt ist, deren Mantelfläche mit Sägezahnrillen zur Spitze hin ausgeführt ist und durch Längsschlitze 22 in Biegefedern (21) unterteilt ist, wobei die Hülse durch eine passende Gegenzahnung im zweiten Schenkel (6) am Rückwärtsgleiten gehindert ist.

7. Verankerung nach Anspruch 6, dadurch gekennzeichnet, dass die Biegefedern im zweiten Schenkel (6) nach dem Festsetzen des Bandes (1) durch einen Sicherungsstift am Zurückfedern in Richtung zur Achse (9) gehindert sind.

## Claims

1. An anchor for a synthetic ligament (1) which is passed through a bone (2), whereby the anchor comprises a housing (3) supported in the bone (2) and a clamping element (4) having a projectile shape (18), and where a ligament (1) is retractable in one shank (5) of housing (3), whilst clamping element (4) is adjustable transversly to same, so as to apply a clamping force on retracted ligament (1), where the housing (3) is constructed outwardly as a Y-tube, whose shanks (5,6) are arranged at a pointed angle (7) of between 40° and 20° to one another, the tube inner surfaces of the first shank (5) for the ligament and of the second shank (6) for the clamping element (4) mutually penetrate along the whole length of the branching, and both the clamping element (4) and the first shank (5) have grooves (8,15,20) transversly to the ligament (1), which jointly encircle and press the ligament (4) by 360° along a length of more than the inner diameter of the first shank (5), whereby the clamping results with a side surface of the elongated projectile shape (18).

2. An anchor according to claim 1, characterised in that measured in the plane of angle (7), the end face of housing (3) has an inclined angle (23) of less than 80° to the first shank (5).

3. An anchor according to claims 1 & 2, characterised in that one of the two shanks (5,6) is extended in its direction via the intersecting point of the two shank axes (9,11).

4. An anchor according to claim 3, characterised in that at least the extended shank has a projecting flange (13) on its outer side in a plane (12) of the end surface of housing (3), for support on the outer side (14) of the bone.

5. An anchor according to any of claims 1-4, characterised in that clamping element (4) is a clamping screw (10) which is continuously adjustable though rotation in the second shank (6), having a cartridge-shaped, grooved peak.

6. An anchor according to any of claims 1-4, characterised in that clamping element (4) is arranged behind a cartridge-shaped peak as a slotted housing, the shell of which is provided with saw-toothed grooves towards the peak and is divided into flexible springs (21) by longitudinal slots (22), where the housing is prevented from back-sliding by appropriate opposing teeth in the second shank.

7. An anchor according to claim 6, characterised in that the flexible springs in the second shank (6) are prevented from springing back in the direction of axis (9) after securing the ligament (1) by means of a locking pin.

## Revendications

1. Ancrage pour un ligament artificiel (1) qu'on a fait passer à travers un os (2), où l'ancrage est constitué d'un manchon (3) supporté dans l'os et d'un élément de serrage (4) d'une forme en projectile (18), et où est insérable dans une branche (5) du manchon (3) un ligament (1) tandis que transversalement à celui-ci, l'élément de serrage (4) est ajustable pour appliquer une force de serrage à la bande enfilée (1), où le manchon (3) est réalisé vers l'extérieur comme tuyau-culotte, dont les branches (5, 6) s'étendent selon un angle aigu (7) entre 40° et 20°, les faces internes de tuyau de la première branche (5) pour le ligament et de la deuxième branche (6) pour l'élément de serrage (4) s'interpénétrant mutuellement sur toute la longueur de la ramification et à la fois l'élément de serrage (4) et la première branche (5) présentent des rainures (8, 15, 20) transversalement au ligament (1) qui entourent et compriment le ligament (4) ensemble sur une longueur supérieure au diamètre interne de la première branche (5) sur 360°, où le serrage a lieu avec une face latérale de la forme de projectile allongée (18).

2. Ancrage selon la revendication 1, caractérisé en ce que, mesuré dans le plan de l'angle (7), le côté frontal du manchon (3) présente un angle d'inclinaison (23) inférieur à 80° à la première branche (5).

3. Ancrage selon la revendication 1 ou 2, caractérisé en ce que l'une des deux branches (5, 6) est prolongée au-delà de l'intersection des deux axes de branche (9, 11) dans sa direction.

4. Ancrage selon la revendication 3, caractérisé en ce qu'au moins la branche prolongée présente à son côté externe dans un plan (12) de la face frontale du manchon (3) une patte saillante (13) pour l'appui au côté externe (14) de l'os.

5. Ancrage selon l'une des revendications 1 à 4, caractérisé en ce que l'élément de serrage (4) est une vis de serrage (10) ajustable dans la deuxième branche (6) progressivement par rotation, avec une pointe rainurée en forme de cartouche.

6. Ancrage selon l'une des revendications 1 à 4, caractérisé en ce que l'élément de serrage (4) derrière une pointe en forme de cartouche, est réalisé comme manchon fendu, dont la face d'enveloppe est réalisée avec des rainures en dents de scie vers la pointe et est subdivisée par des fentes longitudinales (22) en des ressorts spirale (21), où le manchon est empêché, par une contre-denture adaptée dans la deuxième branche (6) de glisser en arrière.

7. Ancrage selon la revendication 6, caractérisé en ce que les ressorts spirale dans la deuxième branche (6), après la fixation du ligament (1), sont empêchés par une broche de sécurité de rebondir dans la direction de l'axe (9).
